# EUROPÄISCHE PATENTSCHRIFT

(11) **EP 1 543 889 B1**
(45) Veröffentlichungstag und Bekanntmachung des Hinweises auf die Patenterteilung: **31.10.2007**
(21) Anmeldenummer: 04027505.9
(22) Anmeldetag: 19.11.2004
(51) Int. Cl.: B08B 3/00, A61L 2/00

(54) **Reinigungs- und Desinfektionsmaschine**
Cleaning and desinfection machine
Machine de nettoyage et de désinfection

(30) Priorität: 15.12.2003 DE 10358999
(43) Veröffentlichungstag der Anmeldung: 22.06.2005
(73) Patentinhaber: BHT Hygienetechnik GmbH, 86368 Gersthofen (DE)
(72) Erfinder: Annecke, Karl Heinz, Dr., 64625 Bensheim (DE)
(74) Vertreter: von Bülow, Tam

(56) Entgegenhaltungen:
- EP-A- 0 890 337
- WO-A-92/11875
- US-A- 5 711 921

## Beschreibung

Die Erfindung bezieht sich auf eine Reinigungs- und Desinfektionsmaschine gemäß Patentansprüch 1.

Eine solche Maschine ist aus der EP 0 890 337 A2 die eine Reinigungs-und Desinfektionmachine gemäß dem oberbegriff des Anspruch 1 offenbart, bekannt. Sie wird auch von der Anmelderin unter der Bezeichnung "INNOVA" seit längerer Zeit vertrieben.

Insbesondere bezieht sich die Erfindung auf eine Reinigungs- und Desinfektionsmaschine für medizinische Geräte, die gereinigt und anschließend desinfiziert werden sollen. Zu diesem Zwecke werden unterschiedliche Reinigungsflüssigkeiten und Desinfektionsflüssigkeiten verwendet, die in auswechselbaren Behältern angeliefert werden. Über Sauglanzen wird die jeweilige Flüssigkeit programmgesteuert aus den Behältern gesaugt und dem Waschraum der Maschine zugeführt.

Trotz unterschiedlicher farblicher Markierungen der Behälter kommt es in der Praxis immer wieder vor, daß die Behälter verwechselt werden und an die falsche Sauglanze angeschlossen werden, was zur Folge hat, daß die medizinischen Geräte, wie z.B. Endoskope, nicht korrekt gereinigt und desinfiziert werden. So ist beispielsweise ein Fall bekannt geworden, bei dem zwei Behälter mit Reinigungsflüssigkeit angeschlossen wurden und damit keiner mit Desinfektionsflüssigkeit, so daß ein Endoskop zwar mehrfach gereinigt aber nicht desinfiziert wurde.

Um Verwechslungen zu vermeiden, wäre es denkbar, Behälter mit unterschiedlichen Einführöffnungen für Sauglanzen zu versehen und an der Maschine unterschiedliche Sauglanzen anzubringen, so daß jeweils eine Sauglanze nur in den "reichtigen" Behälter einführbar ist. Dies hätte aber den gravierenden Nachteil, daß die gesamte Behälterproduktion umgestellt werden müßte, was zu wesentlichen Kostensteigerungen führen würde.

Aus der US-A-5 711 921 ist eine Reinigungs- und Sterilisationsmaschine für medizinische Geräte bekannt, die ebenfalls mehrere Behälter mit unterschiedlichen Reinigunge- und Desinfektionsflüssigkeiten hat, die programmgesteuert selektiv dem zu reinigenden Gegenstand zugeführt werden kann. Zur eindeutigen Identifizierung der Behälter und deren Inhalt hat jeder Behälter einen elektronischen Chip, der mit einer Steuerung der Maschine kommuniziert.

Die WO 92/11875 A1 zeigt eine Sterilisationsmaschine für medizinische Geräte, die ebenfalls mehrere Behälter für unterschiedliche Reinigungs- und Desinfektionsflüssigkeiten aufweist. Jeder Behälter hat einen spezifischen Bajonettverschluß, um Verwechslungen der Behälter zu vermeiden.

Aufgabe der Erfindung ist es daher, die Reinigungs- und Desinfektionsmaschine der eingangs genannten Art dahingehend zu verbessern, daß Verwechslungen der austauschbaren Behälter praktisch ausgeschlossen sind. Diese Aufgabe wird durch die im Patentanspruch 1 angegebenen Merkmale gelöst. Vorteilhafte Ausgestaltungen und Weiterbildungen der Erfindung sind den Unteransprüchen zu entnehmen.

Die Erfindung basiert auf dem Grundgedanken, den in der Sauglanze ohnehin vorhandenen elektrischen Schwimmerschalter, der an die Steuerung der Maschine meldet, ob noch Flüssigkeit in dem Behälter ist, in Verbindung mit einer Steckverbindung zur Identifizierung des Behälters zu verwenden. Je nach Flüssigkeit in dem Behälter wird eine unterschiedliche Steckverbindung verwendet, die mit anderen Steckverbindungen nicht kompatibel ist. Ein Teil der Steckverbindung ist dabei der jeweiligen Sauglanze zugeordnet und der andere Teil dem jeweiligen Behälter. Der dem Behälter zugeordnete erste Teil der Steckverbindung ist beispielsweise über ein Verbindungskabel fest mit dem Behälter verbunden, der zweite Teil mit einer elektrischen Leitung, die von dem Schwimmerschalter zur Steuerung der Maschine führt. Durch diesen zweiten Teil der Steckverbindung ist mindestens eine Ader der elektrischen Leitung von dem Schwimmerschalter zur Steuerung unterbrochen, solange die Steckverbindung nicht gekuppelt ist. Die Steuerung der Maschine erhält dann ein Fehlersignal und startet den Reinigungs- und Desinfektionsvorgang nicht.

An der Steckverbindung wird somit eine künstlich herbeigeführte Unterbrechung hervorgerufen, die durch die als elektrische Brücke wirkende Verbindungsleitung wieder aufgehoben werden kann.

Wenn es sich bei der speziellen Maschine nur um zwei Behälter mit verschiedenen Flüssigkeiten handelt, so kann an einer Sauglanze ein Stecker und am zugeordneten Behälter eine Muffe und an der anderen Sauglanze eine Muffe und am zugeordneten Behälter ein Stecker angebracht sein, so daß vom Aufbau her die identische Steckverbindung verwendet werden kann und lediglich Stecker und Muffe vertauscht sind. Der Vorteil dieser Ausführung liegt darin, daß die identische Stecker-/Muffe-Kombination verwendet werden kann.

Bei mehr als zwei zu unterscheidenden Behältern müssen unterschiedliche Stecker-/Muffe-Kombinationen verwendet werden, die untereinander nicht kompatibel sind.

Nach einer Weiterbildung der Erfindung können an dem jeweiligen Behälter zusätzliche Identifikationsmerkmale angebracht sein, wie z.B. ein Mikrochip oder ein Transponder, der durch die Verbindung mit der Sauglanze durch einen Stromfluß aktiviert wird und dann an die Steuerung der Maschine zusätzliche Informationen über die verwendete Flüssigkeit, wie z.B. Herstellungsdatum, Verfalldatum, Abfülldatum usw. überträgt. Dies kann drahtlos oder drahtgebunden erfolgen. Auf diesem Wege kann dann auch durch entsprechende Kennung des individuellen Behälters verhindert werden, daß dieser unerlaubterweise wieder, nachdem er wieder mit Flüssigkeit befüllt worden ist, ein zweites Mal mit der Sauglanze der Maschinen verbunden wird. Dies stellt einen weiteren Sicherungsschritt gegen die absichtliche mißbräuchliche Wiederbefüllung oder Verwendung von Fremdchemikalien in der Maschine dar.

Mit der Erfindung werden folgende Vorteile erreicht:

Eine Verwechslung von Behältern ist praktisch ausgeschlossen und führt auf alle Fälle zu einem Stillstand der Maschine; bisherige einheitliche Behälter können weiter verwendet werden;
das Nachrüsten vorhandener Maschinen an den Sauglanzen und vorhandener Behälter ist leicht möglich;
der Betrieb der Maschine wird automatisch verhindert, solange nicht das entsprechende Paßstück des Steckverbinders des Behälters mit der Sauglanze verbunden ist;
zusätzlich können nach der oben genannten Weiterbildung der Erfindung große Datenmengen mit weiteren Informationen über die eingesetzte Flüssigkeit zur Maschine übertragen werden.

Im folgenden wird die Erfindung anhand eines Ausführungsbeispieles im Zusammenhang mit der Zeichnung ausführlicher erläutert. Es zeigt:

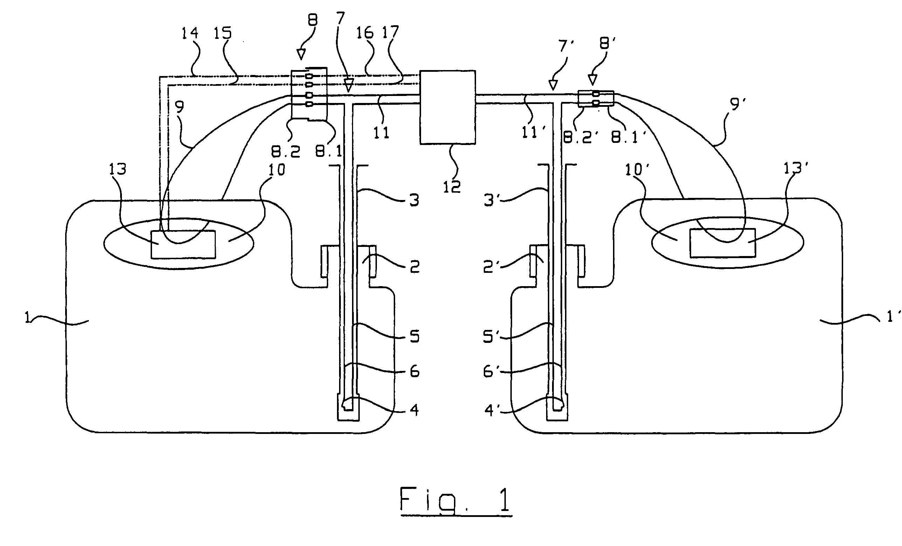

- Fig. 1: eine Prinzipskizze von zwei Behältern mit Sauglanzen und den Steckverbindern.

Ein Behälter 1 hat eine Entnahmeöffnung 2, in die eine Sauglanze 3 einführbar ist. Die Sauglanze 3 hat einen elektrischen Schwimmerschalter 4, der bei vollem Behälter 1 geschlossen ist und dann öffnet, wenn der Flüssigkeitspegel in dem Behälter 1 unter einen bestimmten Wert gesunken ist. Die beiden Kontakte des Schwimmerschalters 4 sind über Adern 5 bzw. 6 einer elektrischen Leitung mit einer Steuerung 12 der Maschine verbunden. Die Ader 6 ist an einer Verzweigungsstelle 7 jedoch unterbrochen und mit einem Anschluß eines Steckverbinders 8 verbunden. Der Steckverbinder 8 kann zweipolig ausgeführt sein und hat ein Steckerteil 8.1 und ein Muffenteil 8.2. Ein Anschluß des Steckerteiles 8.1 ist mit der Ader 6 und damit mit dem Schwimmerschalter 4 verbunden. Der andere Anschluß des Steckerteiles 8.1 ist über eine elektrische Leitung mit dem Steuergerät 12 der Maschine verbunden. Die beiden Anschlüsse des Muffenteiles 8.2 sind über eine elektrische Verbindungsleitung 9 miteinander verbunden, die ihrerseits mechanisch fest mit dem Behälter 1 verbunden ist, beispielsweise durch eine Grifföffnung 10 des Behälters geführt ist. Sind das Steckerteil 8.1 und das Muffenteil 8.2 miteinander gekoppelt, so verbindet die elektrische Leitung 9 die beiden Leitungen 6 und 11 miteinander, womit im Ergebnis der Schwimmerschalter 4 dann korrekt zweipolig mit der Steuerung 12 verbunden ist. Der Steckverbinder 8 mit der elektrischen Leitung 9 wirkt somit als elektrischer Schalter bzw. Brücke, der bzw. die die Verbindung zwischen dem Schwimmerschalter 4 und der Steuerung 12 im gekuppelten Zustand des Steckverbinders 8 herstellt und im entkuppelten Zustand unterbricht.

Ein zweiter Behälter 1' mit Entnahmeöffnung 2' ist identisch aufgebaut und wird über eine Sauglanze 3' mit Schwimmerschalter 4', elektrischen Leitungen 5' und 6' in gleicher Weise mit einem Steckverbinder 8' und einer elektrischen Leitung 9' an die Steuerung 12 angeschlossen.

Der einzige Unterschied besteht in dem Steckverbinder 8', der mit dem Steckverbinder 8 des ersten Behälters 1 nicht kompatibel ist. Somit ist jedem Paar von Behälter 1 bzw. 1' und Sauglanze 3 bzw. 3' je ein eigener unterschiedlicher Steckverbinder 8 bzw. 8' zugeordnet, so daß beispielsweise die Sauglanze 3 für den ersten Behälter 1 nur an diesen angeschlossen werden kann und nicht an den zweiten Behälter 1' und umgekehrt.

Über die Steckverbinder 8 und 8' wird somit der jeweilige Behälter indirekt identifiziert, so daß Verwechslungen ausgeschlossen sind.

Die unterschiedlichen Steckverbinder können auf verschiedene Weise realisiert werden. Im einfachsten Fall von nur zwei Behältern 1 und 1' und zwei Sauglanzen 4 und 4' kann vorgesehen sein, daß das Steckerteil 8.1 für den einen Behälter 1 an der Sauglanze und das Muffenteil 8.2 am Behälter 1 angebracht sind, während an der zweiten Sauglanze 3' das Muffenteil 8.2 und am zweiten Behälter 1' das Steckerteil 8.1 angebracht sind. Bei einer Vertauschung würden sich dann zwei Muffenteile 8.2 und 8.2' und zwei Steckerteile 8.1 und 8.1' gegenüberstehen, die jeweils nicht miteinander koppelbar sind.

Nach einer anderen Variante der Erfindung, die auch für mehr als zwei Behälter und Sauglanzen geeignet ist, sind die Steckerteile mechanisch unterschiedlich ausgeführt derart, daß immer nur ein Paar von Steckerteil und Buchsenteil miteinander koppelbar sind. Dies kann durch unterschiedliche Durchmesser, unterschiedliche Formgebung oder unterschiedliche Anordnung der miteinander zu verbindenden Kontakte erfolgen.

Nach einer Weiterbildung der Erfindung kann jedem Behälter 1 und 1' ein Mikrochip oder Transponder 13 bzw. 13' zugeordnet sein, der durch einen Stromfluß durch die Leitungen 9 bzw. 9' aktiviert wird und dann drahtgebunden oder drahtlos Daten an die Steuerung 12 überträgt. Diese Daten können zusätzliche Informationen über die im Behälter befindliche Flüssigkeit, das Herstellungsdatum, das Verfallsdatum, das Abfülldatum, die Chargennummer, eine Behälternummer oder ähnliches enthalten. Hierdurch kann jeder individuelle Behälter eine individuelle Kennung erhalten, die an die Steuerung 12 der Maschine übertragen wird. Damit kann auch unterbunden werden, daß der Behälter unerlaubterweise nachgefüllt und ein zweites Mal mit der zugeordneten Sauglanze verbunden wird, da in der Steuerung gespeichert sein kann, daß dieser Behälter schon einmal an die Maschine angeschlossen war. Dies stellt einen zusätzlichen Sicherungsschritt gegen die mißbräuchliche Wiederbefüllung oder Verwendung von Fremdchemikalien dar.

Bei einer drahtgebundenen Übermittlung von dem Mikrochip an die Steuerung kann die Ankopplung auch über die Steckverbinder 8 bzw. 8' erfolgen. Wie im Ausführungsbeispiel der Fig. 1 am Behälter 1 durch gestrichelte Linien dargestellt, kann dann der Steckverbinder 8 mehr als zweipolig ausgebildet sein, beispielsweise vierpolig, wobei Datenleitungen 14 und 15 von dem Mikrochip 13 über den Steckverbinder 8 mit Datenleitungen 16 und 17 verbunden werden, die an die Steuerung 12 angeschlossen sind.

## Patentansprüche

1. Reinigungs- und Desinfektionsmaschine mit mindestens zwei auswechselbaren Behältern (1; 1') für unterschiedliche Flüssigkeiten und je einer an die Behälter anschließbaren Sauglanze (3; 3'),
**dadurch gekennzeichnet,**
**daß** jede Sauglanze einen elektrischen Schalter (4; 4') aufweist, der über eine elektrische Leitung mit einer steuerung (12) der Maschine verbindbar ist, wobei in die elektrische Leitung (5, 6; 5', 6') ein Steckverbinder (8, 8') zwischengeschaltet ist, der die Verbindung zwischen dem elektrischen Schalter (4, 4') und der Steuerung (12) herstellt,
**daß** eine Komponente (8.2; 8.1') des Steckverbinders (8; 8') über eine elektrische Verbindungsleitung (9; 9') mit einem Behälter (1; 1') und die andere Komponente (8.1; 8.2') mit dem elektrischen Schalter (4; 4') einer Sauglanze (3; 3') verbunden ist und
**daß** jedem Paar von Behältern (1; 1') und Sauglanzen (3; 3') ein unterschiedlicher Steckverbinder (8; 8') zugeordnet ist.

2. Reinigungs- und Desinfektionsmaschine nach Anspruch 1, **dadurch gekennzeichnet,**
**daß** bei nur zwei Behältern (1; 1') einem ersten Behälter (1) ein Muffenteil (8.2) und dem zweiten Behälter ein Steckerteil (8.1') und der jeweiligen Sauglanze (3; 3') das entsprechende Gegenstück (8.1; 8.2') zugeordnet sind.

3. Reinigungs- und Desinfektionsmaschine nach Anspruch 1, **dadurch gekennzeichnet,**
**daß** die Steckverbinder (8; 8') unterschiedliche Geometrie aufweisen, so daß jede Komponente jedes Steckverbinders nur mit einem zugeordneten Gegenstück verbindbar ist.

4. Reinigungs- und Desinfektionsmaschine nach Anspruch 3, **dadurch gekennzeichnet,**
**daß** die unterschiedlichen Steckverbinder (8; 8') unterschiedlichen Durchmesser, unterschiedliche Form oder eine unterschiedliche Anordnung von Kontakten aufweisen.

5. Reinigungs- und Desinfektionsmaschine nach einem der Ansprüche 1 bis 4, **dadurch gekennzeichnet,**
**daß** die elektrische Verbindungsleitung (9; 9') mechanisch fest mit dem zugeordneten Behälter (1; 1') verbunden ist.

6. Reinigungs- und Desinfektionsmaschine nach einem der Ansprüche 1 bis 5, **dadurch gekennzeichnet,**
**daß** jedem Behälter (1; 1') ein Mikrochip (13; 13') zugeordnet ist, der durch Kopplung des dem jeweiligen Behälter (1; 1') zugeordneten Steckverbinders (8; 8') aktiviert wird und Daten an die Steuerung (12) überträgt.

7. Reinigungs- und Desinfektionsmaschine nach Anspruch 6, **dadurch gekennzeichnet,**
**daß** der Mikrochip (13) über zusätzliche elektrische Leitungen (14, 15, 16, 17) und den zugeordneten Steckverbinder (8) mit der Steuerung (12) verbindbar ist.

8. Reinigungs- und Desinfektionsmaschine nach Anspruch 6, **dadurch gekennzeichnet,**
**daß** der Mikrochip (13) über die elektrische Verbindungsleitung (9, 9') und den zugeordneten Steckverbinder (8) mit der Steuerung (12) verbindbar ist.

9. Reinigungs- und Desinfektionsmaschine nach Anspruch 6, **dadurch gekennzeichnet,**
**daß** der Mikrochip (13) drahtlos mit der Steuerung (12) verbindbar ist, wobei die Verbindung über elektromagnetische Wellen erfolgt.

10. Reinigungs- und Desinfektionsmaschine nach einem der Ansprüche 6 bis 9, **dadurch gekennzeichnet,**
**daß** der Mikrochip ein Transponder (13) ist.

11. Reinigungs- und Desinfektionsmaschine nach einem der Ansprüche 6 bis 10, **dadurch gekennzeichnet,**
**daß** die Steuerung (12) einen Speicher aufweist, der die von dem Mikrochip (13) übermittelten Daten speichert und bei einem Wiederanschließen eines zuvor schon einmal angeschlossenen Behälters (1) den Betrieb der Maschine unterbindet.

## Claims

1. Cleaning and disinfection machine with at leat two exchangeable containers (1; 1') for different liquids and a suction lance (3; 3') for each of the containers to which it is connectable,
**characterized in that**
each suction lance has an electrical switch (4; 4') that is connectable via an electrical line to a controller (12) of the machine,
wherein a plug connector (8, 8') is interposed into the electrical line (5, 6; 5', 6'), said plug connector is making the connection between the electrical switch (4, 4') and the controller (12),
**in that** one component (8.2; 8.1') of the plug connector (8; 8') is connected via an electrical connection line (9; 9') with a container (1; 1') and the other component (8.1; 8.2') with the electrical switch (4; 4') of a suction lance (3; 3') and
**in that** each pair of containers (1; 1') and suction lances (3; 3') is assigned a different plug connector (8; 8').

2. Cleaning and disinfection machine according to claim 1, **characterized in that**
for only two containers (1; 1') a socket part (8.2) is assigned to a first container (1) and a plug part (8.1') is assigned to the second container, and the corresponding counterpiece (8.1; 8.2') is assigned to the associated suction lance (3; 3').

3. Cleaning and disinfection machine according to claim 1, **characterized in that**
the plug connectors (8; 8') have different geometries, so that each component of each plug connector can be connected only to an associated counterpiece.

4. Cleaning and disinfection machine according to claim 3, **characterized in that**
the different plug connectors (8; 8') have different diameters, different shapes, or a different arrangement of contacts.

5. Cleaning and disinfection machine according to one of claims 1 to 4, **characterized in that**
the electrical connection line (9; 9') is permanently joined mechanically to the associated container (1; 1') .

6. Cleaning and disinfection machine according to one of claims 1 to 5, **characterized in that**
a microchip (13; 13') is assigned to each container (1; 1'), said microchip is activated by coupling the plug connector (8; 8') assigned to the associated container (1; 1') and which transmits data to the controller (12).

7. Cleaning and disinfection machine according to claim 6, **characterized in that**
the microchip (13) can be connected via additional electrical lines (14, 15, 16, 17) and the associated plug connector (8) to the controller (12).

8. Cleaning and disinfection machine according to claim 6, **characterized in that**
the microchip (13) can be connected via the electrical connection line (9, 9') and the associated plug connector (8) to the controller (12).

9. Cleaning and disinfection machine according to claim 6, **characterized in that**
the microchip (13) can be connected to the controller (12) in a wireless manner, wherein the connection being realized by means of electromagnetic waves.

10. Cleaning and disinfection machine according to one of the claims 6 to 9, **characterized in that**
the microchip is a transponder (13).

11. Cleaning and disinfection machine according to one of the claims 6 to 10, **characterized in that**
the controller (12) comprises a memory which stores data transmitted from the microchip (13) and prevents the operation of the machine, if a container (1) already connected previously is reconnected.

## Revendications

1. Machine de nettoyage et de désinfection dotée d'au moins deux bacs échangeables (1, 1') destinés à différents liquides et d'une lance aspirante (3, 3') pouvant être raccordée à chaque bac,
**caractérisée**
**en ce que** chaque lance aspirante présente un interrupteur électrique (4 ; 4'), qui peut être connecté par une ligne électrique à un dispositif de commande (12) de la machine, de sorte qu'un connecteur à fiches (8, 8') est intercalé dans la ligne électrique (5, 6, ;5', 6'), lequel réalise la liaison entre l'interrupteur électrique (4, 4') et le dispositif de commande (12),
**en ce qu'**un composant (8.2; 8.1') du connecteur à fiches (8 ; 8') est connecté par l'intermédiaire d'une ligne de liaison électrique (9, 9') à un bac (1 ; 1') et les autres composants (8.1, 8.2') sont reliés à l'interrupteur électrique (4, 4') d'une lance aspirante (3, 3') et
**en ce qu'**un connecteur à fiches (8, 8') différent correspond à chaque paire de bacs (1, 1') et de lances aspirantes (3, 3').

2. Machine de nettoyage et de désinfection selon la revendication 1, **caractérisée en ce que** pour une conception avec uniquement deux bacs (1, 1'), une partie de manchon (8.2) correspond à un premier bac (1) et une partie de fiche (8.1') correspond au second bac et la pièce opposée appropriée (8.1, 8.2') correspond à la lance d'aspiration appropriée (3, 3').

3. Machine de nettoyage et de désinfection selon la revendication 1, **caractérisée en ce que** les connecteurs à fiches (8, 8') présentent différentes géométries, de sorte que chaque composant de chaque connecteur à fiches puisse être uniquement relié à une pièce opposée correspondante.

4. Machine de nettoyage et de désinfection selon la revendication 3, **caractérisée en ce que** les différents connecteurs à fiches (8, 8') présentent un diamètre différent, une forme différente ou un agencement différent des contacts.

5. Machine de nettoyage et de désinfection selon l'une quelconque des revendications 1 à 4, **caractérisée en ce que** la ligne de liaison électrique (9 ; 9') est reliée solidement et mécaniquement au bac correspondant (1; 1 1').

6. Machine de nettoyage et de désinfection selon l'une quelconque des revendications 1 à 5, **caractérisée en ce qu'**une puce électronique (13, 13') correspond à chaque bac (1 ; 1'), laquelle est activée par le couplage du connecteur à fiches (8 ; 8') correspondant à chaque bac (1 ; 1') et transmet des données au dispositif de commande (12).

7. Machine de nettoyage et de désinfection selon la revendication 6, **caractérisée en ce que** la puce électronique (13) peut être connectée au dispositif de commande (12) par des lignes électriques supplémentaires (14, 15, 16, 17) et par le connecteur à fiches (8) correspondant.

8. Machine de nettoyage et de désinfection selon la revendication 6, **caractérisée en ce que** la puce électronique (13) peut être connectée au dispositif de commande (12) par la ligne de liaison électrique (9, 9') et le connecteur à fiches (8) correspondant.

9. Machine de nettoyage et de désinfection selon la revendication 6, **caractérisée en ce que** la puce électronique (13) peut être reliée sans fil au dispositif de commande (12), ainsi la liaison est réalisée par l'intermédiaire d'ondes électromagnétiques.

10. Machine de nettoyage et de désinfection selon l'une quelconque des revendications 6 à 9, **caractérisée en ce que** la puce électronique est un transpondeur (13).

11. Machine de nettoyage et de désinfection selon l'une quelconque des revendications 6 à 10, **caractérisée en ce que** le dispositif de commande (12) présente une mémoire, qui stocke les données transmises par la puce électronique (13) et qui interrompt le fonctionnement de la machine lors d'un nouveau raccordement d'un bac (1) ayant déjà été une fois raccordé.
